# EUROPÄISCHE PATENTSCHRIFT

(11) **EP 2 641 553 B1**
(45) Veröffentlichungstag und Bekanntmachung des Hinweises auf die Patenterteilung: **08.10.2014**
(21) Anmeldenummer: 13156972.5
(22) Anmeldetag: 27.02.2013
(51) Int. Cl.: A61B 18/14, A61B 19/00, G01K 11/32, G01B 11/16, A61B 18/24

(54) **Messeinrichtung und Katheteranordnung**
Measuring device and catheter arrangement
Outil de mésure et agencement de cathéter

(30) Priorität: 23.03.2012 US 201261614547 P
(43) Veröffentlichungstag der Anmeldung: 25.09.2013
(73) Patentinhaber: VascoMed GmbH, 79589 Binzen (DE)
(72) Erfinder: Bitzer, Andreas, 8032 Zürich (CH); Fandrey, Stephan, 8910 Affoltern am Albis (CH)
(74) Vertreter: Lindner-Vogt, Karin L.

(56) Entgegenhaltungen:
- US-A- 5 399 854
- US-A1- 2007 060 847
- US-B1- 6 278 810

## Beschreibung

Die Erfindung betrifft eine Katheteranordnung mit einem Katheter, insbesondere Ablationskatheter, mit einem in einen distalen Abschnitt integrierten optischen Kraftsensor, der zur Messung einer auf den distalen Abschnitt wirkenden äußeren Kraft ausgebildet und angeordnet ist.

In bestimmten Einsatzfeldern von Kathetern oder ähnlichen Vorrichtungen, z.B. Elektrodenleitungen, ist eine Andruckkraft an benachbartes Gewebe von Bedeutung für deren Funktion, sodass eine Erfassung dieser Kontaktkraft von Interesse ist. Dies trifft in besonderem Maße für sogenannte Ablationskatheter zu, mit denen Gewebebereiche verödet bzw. Gewebeteile abgetragen werden.

Es ist ein Ablationskatheter ("TactiCath", Fa. Endosense) bekannt, welcher die Messung einer auf das distale Katheterende einwirkenden Kraft - im Anwendungsfall also der erwähnten Kontaktkraft - nach Betrag und Richtung während eines Ablationsvorganges ermöglicht. Dieser Katheter nutzt das Prinzip des sogenannten FBG (Faser-Bragg-Gitter)-Sensors, wobei drei Fasern mit jeweils einem FBG-Sensor am Faserende die für eine 3D-Kraftmessung benötigte Gruppe von Sensoren bilden, die zur gemeinsamen Messsignalverarbeitung an eine Signalverarbeitungseinheit einschließbar sind. Die Sensoren sind in einem Winkelabstand von 120° außen auf einem verformbaren Zylinder aufgebracht.

In der US 2008/0285909 A1 wird die Funktionsweise von FBG-Sensoren zur Bestimmung von Verdrillungen oder Krümmungen des Katheterkörpers ausführlich beschrieben, und auch die Funktionsweise des vorgenannten Kraftsensors mit mehreren FBG-Fasern auf einem verformbaren Zylinder ist in dieser Druckschrift erläutert.

Es ist, wie in der WO 2009/138957 A2 beschrieben, eine Temperaturkompensation mittels elektrischer Thermoelemente vorgesehen, weil bei dem FBG-Messverfahren bereits kleine Temperaturänderungen bzw. Abweichungen zwischen den einzelnen Sensoren große Messunsicherheiten verursachen können und bei einem elektrothermischen Ablationsvorgang ganz erhebliche Temperaturschwankungen an der Spitze des Ablationskatheters auftreten können. Aus der genannten Druckschrift ist es auch bekannt, zur Verringerung der Auswirkung von Temperaturänderungen auf das Kraftmessergebnis die thermische Leitfähigkeit zwischen einem Ablationskopf eines Ablationskatheters (der sehr heiß werden kann) und den Kraftsensoren zu verringern. In jedem Falle ist der Aufbau des Katheters hochgradig komplex, was zu hohen Produktionskosten für den Katheter selbst und auch für die zugehörigen Auswertungseinheiten führt.

Das optische Messprinzip der FBG-Sensorik ist allgemein sowie insbesondere auch in seiner Anwendung für Kraftmessungen und Temperaturmessungen bekannt; vgl. etwa www.wikipedia.org/wiki/Fiber_Bragg_grating oder A. Othonos, K. Kalli: "Fiber Bragg Gratings: Fundamentels and Applications in Telecommunications and Sensing" Artec House 1999, sowie (speziell bezogen auf Spannungs- und Temperaturmessungen) US 5,399,854. Eine ausführliche Erläuterung des Messprinzips ist daher hier nicht erforderlich.

Aus der unveröffentlichten US-Patentanmeldung Nr. 61/446,053 der Anmelderin sind ein Katheter, eine Katheteranordnung und eine Kraftmesseinrichtung bekannt, bei denen eine in einem Sensorhalter fixierte einzelne FBG-Faser drei Kraftsensorbereiche umfasst, die zur gemeinsamen Messsignalverarbeitung zusammenwirkende Kraftsensoren bilden, und bei der auf der Faser zusätzlich ein T-Sensorbereich vorgesehen sein kann.

US 5 399 854 offenbart einen kombinierten Kraft- und Temperatur sensor auf Basis von Fiber-Bragg-Gratings.

Der Erfindung liegt die Aufgabe zugrunde, eine sowohl in ihrem Aufbau als auch in Bezug auf den Anschluss an eine zugehörige Signalverarbeitungseinheit und auf die Signalverarbeitung vereinfachte und daher kostengünstige Katheteranordnung der eingangs genannten Art anzugeben.

Diese Aufgabe wird durch eine Kraft- bzw. Dämpfungsmesseinrichtung mit den Merkmalen des Anspruchs 1 gelöst. Des Weiteren wird eine entsprechende neuartige Katheteranordnung mit den Merkmalen des Anspruchs 3 bereitgestellt. Zweckmäßige Fortbildungen des Erfindungsgedankens sind Gegenstand der jeweiligen abhängigen Ansprüche.

Die Erfindung geht von der Überlegung aus, das bisherige Konzept des Vorsehens eines zusätzlichen T-Sensors zur Erfassung (und nachfolgenden Kompensation) der signifikanten T-Abhängigkeit des Kraftsignals eines optischen Kraftsensors aufzugeben. Sie schließt weiter den Gedanken ein, stattdessen das Messsignal des optischen Kraftsensors selbst zur Ermittlung relevanter Temperaturänderungen zu nutzen. Weiterhin gehört daraufhin zur Erfindung die Überlegung, das T-Signal vom Kraft-Signal ein und desselben Sensors in geeigneter Weise unterscheidbar zu machen. Als technisches Mittel zur Erzielung dieser Unterscheidung wird bei der Erfindung die Modulation des die Kraft repräsentierenden ersten Messsignalanteils, in Verbindung mit einer differenzierten Auslesung dieses ersten Messsignalanteils und des verbleibenden zweiten, unmodulierten Messsignalanteils, genutzt.

Das Funktionsprinzip lässt sich also wie folgt verstehen:

Durch die Modulation enthält der Sensor-Messwert einen periodischen Anteil bei der Frequenz f mit der Amplitude a, und Phase p und einen zeitlich sich langsam verändernden Offset o. Über die dynamischen Messgrößen, die Amplitude a und die Phase p lässt sich direkt die Dämpfung bestimmen, welche durch eine Einwirkung von außen in das System eingebracht wird. In der Regel erfolgt dies bei einem im Gebrauch befindlichen Katheter über den Anpressdruck des Gewebes an den schwingungsfähigen Messkopf. Die unmodulierte Messgröße, also der Offset o, enthält dagegen die Temperaturinformation des Sensors.

Kommerzielle optische Fiber-Bragg Dehnungsmessstreifen (FBG) können mit Frequenzen bis in den MHz-Bereich, bevorzugt bis zu 2.5 kHz ausgelesen werden. Die Temperatur variiert dagegen im Bereich weniger Hz. Dadurch lassen sich die beiden Messgrößen hervorragend voneinander unterscheiden, dies ermöglicht eine zuverlässige Messung mit nur einem Sensor. Ähnliches gilt für andere optische Dehnungs- bzw. Kraftsensoren, etwa vom Fabry-Perot-Typ.

Die Erfindung erlaubt eine wesentlich vereinfachte und somit in der Herstellung günstigere Lösung als bekannte Kraftmess-Katheteranordnungen, da sie mit nur einem einzigen optischen (FBG-)Sensor sowohl für die Kraft- als auch für die Temperaturmessung auskommt. Zudem ist es denkbar, dass mit der Erfindung eine Gewebeveränderung, welche durch eine zuvor erfolgte Ablationsprozedur verursacht wurde, nachgewiesen werden kann (s. dazu weiter unten). Damit würde sich das Verfahren auch zur anschließenden Diagnose eignen, was bei bisherigen Verfahren nicht möglich ist.

Die Umsetzung des Konzepts wird mit einem auf Resonanz abgestimmten Vibrations-Katheterkopf ermöglicht, welcher an einen FBG Sensor so angebunden ist, dass dieser die Schwingung/ Vibration des Katheterkopfes misst. Dabei soll die Resonanzfrequenz so gewählt sein, dass sie möglichst nahe bei der Modulationsfrequenz liegt. Ferner muss ein ausreichender Wärmekontakt bestehen, um die Temperatur zu ermitteln. Um das zuvor beschriebene schwingungsfähige System zu einer erzwungen Schwingung anregen zu können, bedarf es zudem einer treibenden Anregungskraft welche von außen Energie in das auf Resonanz abgestimmte Schwingungssystem einbringt. Hierfür sind verschiedene Ausführungen denkbar die im Folgenden aufgeführt sind.

In einer Ausführung der Erfindung wird die erwähnte Modulation als Amplitudenmodulation realisiert, wobei der Katheter insbesondere einen mit dem Kraft- und Temperatursensor mechanisch verbundenen Schwingkörper und Mittel zur Übertragung des Auslesesignals zum Schwingkörper und zum Versetzen desselben in Schwingungen mit der Trägerfrequenz umfasst. Der Schwingkörper trägt dem Kraft- und Temperatursensor die Trägerfrequenz auf, während auf den distalen Katheterabschnitt einwirkende Kräfte die y-Kurve des modulierten Messsignals ergeben.

In einer Ausgestaltung umfassen die Mittel zum Übertragen des Auslesesignals einen Antriebsdraht, der bis zum proximalen Ende des Katheters verläuft. Am besagten proximalen Ende des Katheters oder außerhalb desselben (aber verbunden mit dem Antriebsdraht) befindet sich ein Schwingungserzeuger, der den Antriebsdraht in Schwingungen versetzt. Dies kann beispielsweise ein elektromechanischer oder Piezo-Wandler sein.

In einer alternativen Ausgestaltung umfassen die Mittel zum Übertragen des Auslesesignals eine elektrische Leitung, die sich bis zu einem Anschluss am proximalen Ende des Katheters erstreckt sowie einen elektromechanischen oder Piezo-Wandler. Der Wandler ist also hier innerhalb des Katheterkörpers angeordnet, und der bei der vorigen Ausgestaltung vorgesehene Antriebsdraht kann entfallen.

In einer weiteren Ausgestaltung umfassen die Mittel zum Übertragen des Auslesesignals eine sich bis zum proximalen Ende des Katheters erstreckende Flüssigkeitsleitung, insbesondere einen Spülkanal, und einen hydraulischen Wandler. Speziell wenn ein ohnehin im Katheterkörper vorgesehener Spülkanal zur Ausführung der Erfindung genutzt werden kann, lässt sich die Erfindung auf diese Weise ohne großen zusätzlichen konstruktiven Aufwand realisieren.

In einer weiteren Ausgestaltung ist vorgesehen, dass die Mittel zum Übertragen des Auslesesignals eine sich bis zum proximalen Ende des Katheters erstreckende Lichtleitfaser und einen dem Schwingkörper zugeordneten opto-mechanischen Wandler umfassen. Laserpulse, deren Pulsdauer sehr kurz ist (10^-9 - 10^-15 Sekunden) ermöglichen den Übertrag von Lichtpulsen mit einer sehr hohen Impulsdichte. Mittels derartiger Laserpulse ist es grundsätzlich möglich, den auf Resonanz abgestimmten Schwingkörper zu einer erzwungenen Schwingung anzuregen. Eine derartige Ausführung würde ebenfalls den konstruktiven Aufwand im Katheterkopf gering halten. Lediglich eine Faser müsste zusätzlich in den Katheter mit eingebracht werden.

Die Anregung könnte auch durch ein von außen eingestrahltes moduliertes Magnetfeld erfolgen, wodurch auf den schwingungsfähigen Sensorkopf eine Kraft übertragen werden könnte sofern dieser magnetisch wäre. Auch hier wären kaum zusätzliche Komponenten in der Katheterspitze erforderlich.

Zweckmäßige Ausgestaltungen der vorgeschlagenen Kraft- bzw. Dämpfungsmesseinrichtung ergeben sich ohne weiteres aus den obigen Hinweisen zur vorgeschlagenen Katheteranordnung, so dass von Wiederholungen hier Abstand genommen wird. Speziell ergeben sich für einen Katheter mit einer solchen Messeinrichtung Ausgestaltungen der Mittel zum Übertragen des Auslesesignals, wie sie oben erwähnt wurden.

Zur Ausgestaltung der Auslese- und Verarbeitungsmittel der Katheteranordnung bzw. Messeinrichtung ist auf folgendes hinzuweisen:

Insbesondere lässt sich die Kontaktgüte über die Dämpfung bestimmen, welche in ein angeregtes, periodisch oszillierendes System eingebracht wird. Diese kann als Amplitudenabnahme sowie aus der Phasenverschiebung abgeleitet werden und hat keinen Einfluss auf das zeitliche Mittel des Sensorwertes, aus welchem sich die Temperatur ableiten lässt.

Zudem lässt sich aus dem Vergleich zweier Messungen, welche unter gleichen äußeren Umständen erfolgt sind, die sich aber dadurch unterscheiden, dass die eine unmittelbar vor und die andere direkt nach einem Ablationsvorgang durchgeführt worden ist, anhand einer möglichen Veränderung des Dämpfungswertes auf eine Veränderung des Gewebezustandes rückschließen, welche z.B. durch einen Ablationsvorgang erzielt wurde. Diese Möglichkeit beruht darauf, dass Gewebe, welches mittels Ablation verändert wird und insbesondere vernarbt, andere mechanische Eigenschaften aufweist als unbehandeltes Gewebe. Verhärtetes Gewebe wird eine andere Dämpfung hervorrufen als vergleichsweise weiches Gewebe, und somit kann grundsätzlich ermittelt werden, ob eine Ablation erfolgreich war. Insbesondere lässt sich das daran feststellen, dass nach der Ablation (bei unverändertem Anpressdruck des distalen Katheterendes) eine veränderte Dämpfung des Schwingungssystems gemessen wird.

Des Weiteren kommt ein Aspekt hinzu, der mit der Ablationskontrolle zusammenhängt. Mit dem vorgeschlagenen System ist es möglich, die Entstehung von sog. Bubbles - also Luftblasen während der Ablation - vorherzusagen. Diese Bubbles gilt es in jedem Fall zu verhindern. Es hat sich gezeigt, dass kurz vor der Entstehung dieser Bubbles Vibrationen im Katheter entstehen, die durch die erfindungsgemäße Technik erfasst werden können. Vorteile und Zweckmäßigkeiten der Erfindung ergeben sich aus der nachfolgenden Beschreibung von Ausführungsbeispielen der Erfindung anhand der Figuren. Von diesen zeigen:
- Fig. 1: eine schematische Darstellung einer Ausführung der erfindungsgemäßen Katheteranordnung,
- Fig. 2: eine schematische Darstellung einer weiteren Ausführung der erfmdungsgemäßen Katheteranordnung,
- Fig. 3: eine schematische Darstellung einer weiteren Ausführung der erfindungsgemäßen Katheteranordnung,
- Fig. 4: eine schematische Darstellung einer weiteren Ausführung der erfindungsgemäßen Katheteranordnung, und
- Fig. 5A bis 5D: verschiedene Darstellungen erfindungswesentlicher Komponenten, die im distalen Bereich des Katheters einer weiteren Ausführungsform der vorgeschlagenen Katheteranordnung vorgesehen sind.

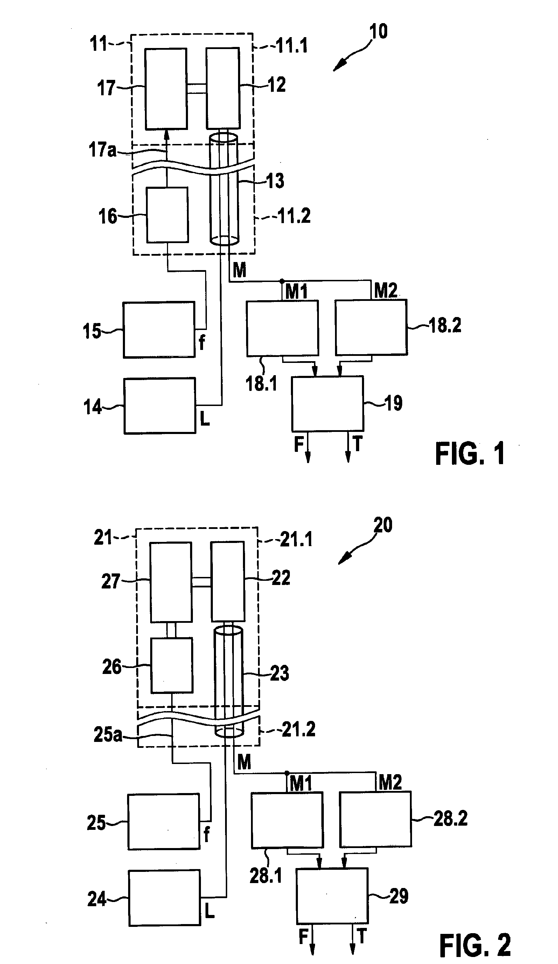

Fig. 1 zeigt die im Zusammenhang mit der Erfindung wesentlichen Teile einer Katheteranordnung 10, die einen Ablationskatheter 11 mit einem Katheterkopf 11.1 und einem Katheterkörper 11.2 umfasst. Im Katheterkopf 11.1 ist ein optischer Kraft- und Temperatursensor 12 angeordnet, an den eine sich bis zum proximalen Ende des Katheterkörpers 11.2 erstreckende Lichtleitfaser 13 angeschlossen ist. Eine Anregungslichtquelle 14 erzeugt und strahlt Messlicht L über die Lichtleitfaser 13 in den optischen Sensor 12 ein, und ein gemäß dem Dehnungszustand und somit gemäß einer auf den optischen Sensor einwirkenden Kraft moduliertes Messsignal M gelangt von dort wiederum über die Lichtleitfaser 13 zu Auswertungsmitteln.

Ein Trägerfrequenzgenerator 15 erzeugt eine elektrische Schwingung f von beispielsweise einigen hundert Hz, die über einen elektromechanischen Wandler 16 am proximalen Ende des Katheterkörpers 11.2 in eine mechanische Schwingung umgewandelt und über einen sich bis in den Katheterkopf erstreckenden Antriebsdraht 17a zu einem Schwingkörper 17 im Katheterkopf 11.1 übertragen wird. Dieser ist mechanisch mit dem optischen Sensor 12 gekoppelt und überträgt seine Schwingungen an diesen, so dass dort ein mit der Frequenz f moduliertes Auslesesignal vorliegt.

Dieses modulierte Auslesesignal bewirkt, dass das Messsignal M zwei Komponenten umfasst, nämlich eine mit der Trägerfrequenz f modulierte Komponente M1 und eine nichtmodulierte (in der Zeitdomäne der Trägerfrequenz als stationär anzusehenden) zweite Komponente M2. Die Messsignalanteile M1, M2 gelangen zu ersten bzw. zweiten Auslesemitteln 18.1 bzw. 18.2, wo sie ausgelesen werden, und schließlich zu einer Verarbeitungseinheit 19, wo eine ausschließlich kraft- bzw. dämpfungsabhängige Komponente F und eine ausschließlich temperaturabhängige Komponente T extrahiert werden.

Eine modifizierte Katheteranordnung 20 gemäß Fig. 2 weist weitgehend den selben Aufbau wie die oben beschriebene Katheteranordnung 10 auf, so dass insoweit an Fig. 1 angelehnte Bezugsziffern verwendet werden und die bereits erläuterten Teile nicht nochmals beschrieben werden. Der wesentliche Unterschied gegenüber der Katheteranordnung 10 besteht darin, dass die Trägerfrequenz f vom Trägerfrequenzgenerator 25 über eine elektrische Leitungsverbindung 25a bis in den Katheterkopf übertragen und erst dort über einen Piezowandler 26 in eine mechanische Schwingung umgewandelt wird. Durch direkte, mechanische Kopplung des Piezowandlers 26 an den Schwingkörper 27 wird die Schwingung auf den letzteren übertragen.

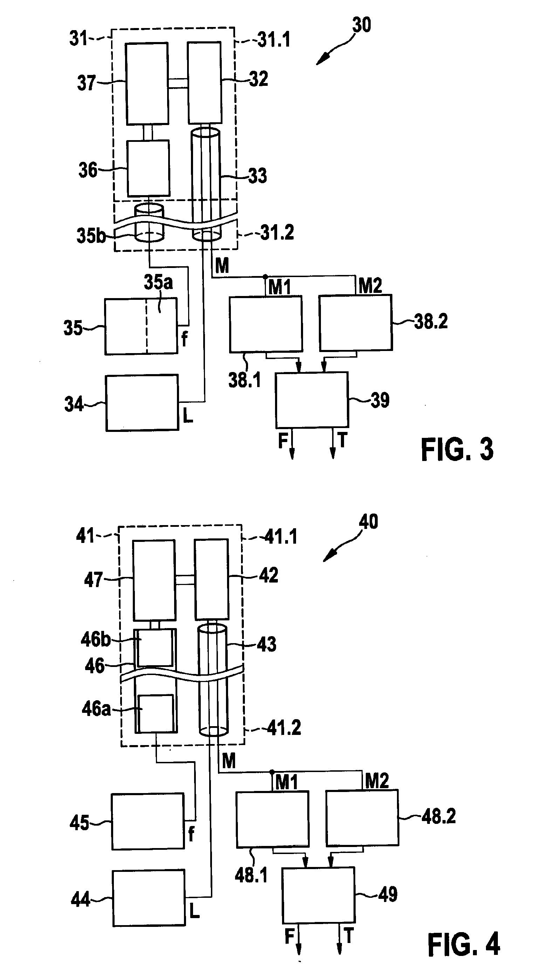

Auch die in Fig. 3 gezeigte Katheranordnung 30 ähnelt den beiden vorgenannten Anordnungen 10 und 20 weitgehend, so dass auch hier an Fig. 1 und 2 angelehnte Bezugsziffern gewählt wurden und die entsprechenden Komponenten - insbesondere diejenigen zur Erzeugung und Übertragung des Anregungslichts und zur Übertragung und Auswertung des Messsignals - hier nicht nochmals erläutert werden. Der wesentliche Unterschied zu den vorbeschriebenen Anordnungen besteht hier darin, dass der Trägerfrequenzgenerator 35 einen optischen Ausgang 35a hat und sein Ausgangssignal in eine Lichtleitfaser 35b eingekoppelt wird, die vom proximalen Ende des Katheterkörpers 31.2 bis zum Katheterkopf 31.1 reicht. Dort befindet sich ein opto-mechanischer Wandler 36, der mechanisch direkt an den Schwingkörper 37 gekoppelt ist und die in eine mechanische Schwingung umgewandelte Frequenz F direkt an jenen überträgt.

Bei der in Fig. 4 gezeigten Anordnung besteht ein Unterschied zu den vorab erläuterten Anordnungen nur in der Übertragung der Auslese- bzw. Trägerfrequenz f zum Schwingungskörper 47. Diese erfolgt hier hydraulisch durch eine Flüssigkeitssäule in einem Spülkanal 46. Dem Spülkanal 46 ist am distalen Ende des Katheters ein elektrohydraulischer Wandler 46a und am distalen Kanalende, mechanisch gekoppelt mit dem Schwingkörper 47, ein hydraulisches Wandler- bzw. Abschlussglied 46b zugeordnet. Der Antrieb des Schwingkörpers 47 erfolgt hier also über eine Flüssigkeitssäule, speziell die Spülflüssigkeit in einem ohnehin im Katheter 41 vorgesehenen Spülkanal.

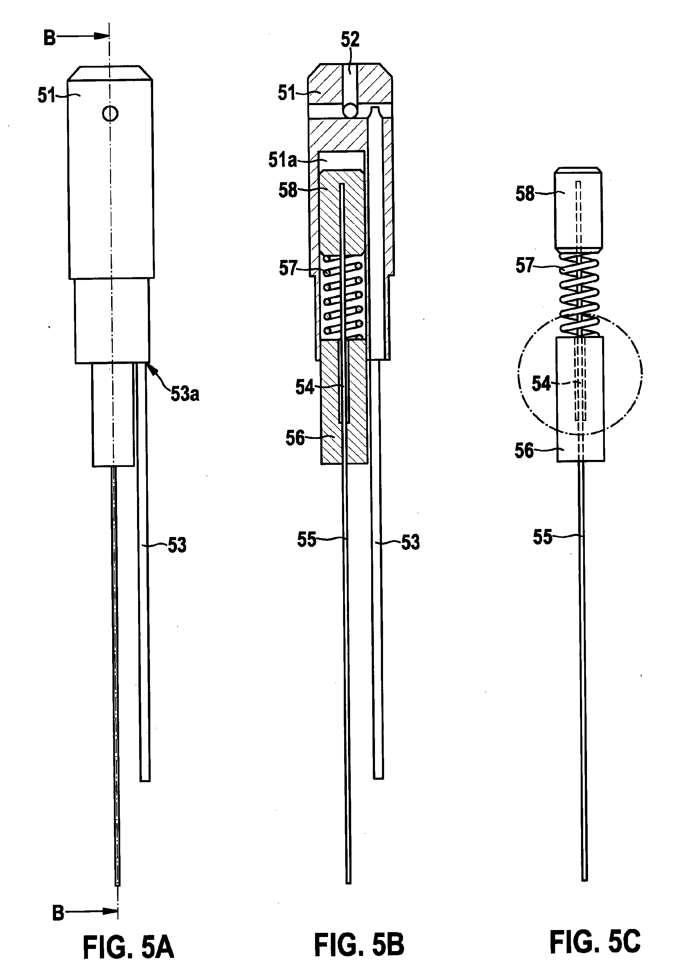

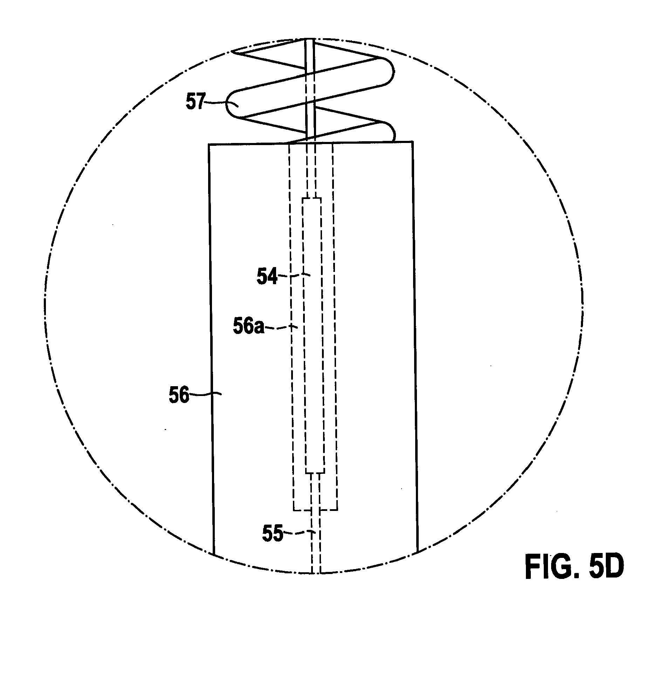

Fig. 5A bis 5D zeigen als im Zusammenhang mit der Erfindung wesentliche Elemente eines Ablationskatheters einen Katheterkopf 51 mit einer Spülkanalanordnung 52, die in der Spitze und am Umfang nahe dem distalen Ende des Katheters mehrere Ausgänge umfasst, und einem am Katheterkopf 51 an einer Befestigungsstelle (Schweißpunkt etc.) 53a befestigten Zugdraht 53.

Als Kraft- bzw. Dämpfungsmesseinrichtung ist im Inneren des Katheterkopfes eine FBG-Dehnungsmesseinrichtung 54 mit angeschlossener Glasfaser 55 zur Übertragung eines Anregungssignals in den FBG-Sensor und zur Übertragung des abgegriffenen Messsignals zum proximalen Katheterende und zu einer Auswertungseinrichtung vorgesehen. An einem Sensorgehäuse 56 ist über ein Federelement 57 ein Schwingkörper 58 angebracht, der zusammen mit dem Sensorgehäuse 56 und dem Federelement in einer zylindrischen Bohrung 51 a des Katheterkopfes sitzt. Eine optional um das FBG-Gitter 54 herum in dem entsprechenden Kanal 56a des Sensorgehäuses 56 vorgesehene Wärmeleitpaste verbessert den thermischen Kontakt zwischen dem Katheterkopf (und somit dessen Gewebs-Umgebung) und dem Gitter und somit dessen Ansprechverhalten und Präzision in seiner Funktion als T-Sensor.

Nach den obigen kurzen Erläuterungen zur Konstruktion verschiedener Ausführungen der vorgeschlagenen Katheteranordnung ist der Katheterkopf des jeweiligen Katheters als schwingungsfähiges System ausgelegt, und eine optische Sensorik, die ein FBG-Sensorelement und eine angeschlossene Lichtleitfaser umfasst, dient zur Messung und Analyse der ihm aufgeprägten Schwingung.

Die Schwingungsanregung kann beispielsweise über den Zugdraht, ein akustisches Signal im Spülschlauch, ein elektrisches Signal in Verbindung mit einem Piezoelement oder einem elektromagnetischen Element, über ein optischen Impuls oder über eine externe Kraft wie beispielsweise ein externes Magnetfeld in das System eingebracht werden. Ziel der Anregung ist es den Katheterkopf in Schwingung zu versetzen. Wenn der Katheterkopf eine Schwingungsbewegung ausführt, so wird der Schwingkörper, welcher über ein Federelement zum Katheterkopf verbunden ist ebenfalls in Schwingung versetzt.

Der Fiber-Bragg-Sensor misst nun die Relativbewegung zwischen Katheterkopf und Schwingkörper. Die Messgrößen sind Amplitude der Bewegung sowie Phase relativ zur Erregeroszillation. Wenn der Katheterkopf nun mit umliegendem Gewebe in Kontakt kommt, so wird eine zusätzliche Dämpfung ins System eingebracht, die die Amplitude und die Phase der Oszillation beeinflusst. Daher lässt sich aus den Messgrößen die Dämpfung und somit die Kontaktgüte des Katheterkopfes zum umliegenden Gewebe ermitteln.

Bei einer Temperaturänderung dehnt sich das System gemäß den Wärmeausdehnungskoeffizienten der verwendeten Materialen aus. Diese Ausdehnung hat eine Längenänderung der Faser zur Folge. Dadurch ändert sich der Gitterabstand des Fiber-Bragg-Gitters, was zu einer spektralen Verschiebung seiner Reflexionseigenschaften führt. Daraus lässt sich die Temperatur ermitteln.

Führt das System gleichzeitig eine Schwingung aus, so verhält sich deren Auslenkung symmetrisch zu einer Nulllage. Der Messwert, welcher der Nulllage entspricht, entspricht genau dem Temperaturmesswert. Durch die Ermittlung des zeitlichen Mittelwertes, lässt sich diese Nulllage (und damit die Temperatur) ermitteln, da sich die Auslenkung der Oszillation immer symmetrisch relativ zur Nulllage verhält. Der zeitliche Mittelwert des FBG-Sensors repräsentiert daher die Temperatur.

Die Anregung bzw. das Messverfahren kann entweder in der Zeit-Domäne oder in der Frequenz-Domäne ausgeführt werden:

### Zeit-Domäne:

Kurze Anregungspulse werden in einer Ausgestaltung des Messverfahrens mit einer Wiederholrate in das System eingebracht, die deutlich größer ist als die Eigenfrequenz des Schwingungssystems. Dadurch lässt sich das zeitliche Abklingverhalten des Amplitudenrückgangs nach der Anregung durch den Puls ermitteln. Aus der Zeitkonstante, die diesen Rückgang charakterisiert, geht direkt der Dämpfungskoeffizient hervor.

### Frequenz-Domäne:

Dabei wird das System kontinuierlich mit einer konstanten Anregungsamplitude mittels einer harmonischen Anregung bei etwa der Eigenfrequenz des Schwingungssystems in Schwingung versetzt. Die Amplitude und Phase welche sich ohne äußere Einwirkungen auf das System ergeben, lassen sich leicht ermitteln, beispielsweise bei einer Referenzmessung außerhalb des Körpers, oder auch im Körper, aber zu einem Zeitpunkt, zu dem kein Anpressdruck an das Gewebe gegeben ist. Wird nun der Katheter mit Gewebe in Kontakt gebracht, so wird das System gedämpft und ein Amplitudenrückgang sowie eine Phasenverschiebung zwischen Erregeroszillation und der des Schwingungssystems gemessen. Daraus lässt sich unmittelbar die Dämpfung ermitteln.

Bei dieser Variante ist es auch denkbar, dass man Messfilter wie LockIn-Verstärker einsetzt, die eine extrem hohe Messsensitivität aufweisen. Bei einem derartigen Verfahren würde man das Erregersignal als Referenzsignal am LockIn-Verstärker anlegen und an dessen Eingang das Messsignal, welches vom FBG-Sensor kommt. Der LockIn-Verstärker multipliziert nun den Eingang mit seinem Referenzsignal und integriert das Ergebnis über eine bestimmte Zeitspanne, wodurch alle Frequenzen gefiltert werden, außer der Erregerfrequenz. Dies ermöglicht ein extrem gutes Signal/Rausch-Verhältnis. Als Ausgabewert erhält man dann direkt den Wert der gemessenen Amplitude des FBG-Sensors, sowie die Phase relativ zur Erregerschwingung.

## Patentansprüche

1. Messeinrichtung für Kraft- und/oder Dämpfungsmessungen (54), mit einem einzelnen kombinierten optischen Kraft- und Temperatursensor (12; 22; 32; 42; 54) und Eingangsmitteln (16, 17a; 25a, 26; 35b, 36; 46a, 46, 46b; 53) zur Einspeisung eines mit einer Trägerfrequenz modulierten Auslesesignals und Ausgangsmitteln (13; 23; 33; 43; 55) zur Ausgabe eines Messsignals, welches einen mit der Trägerfrequenz modulierten ersten Messsignalanteil (M1) und einen nicht mit der Trägerfrequenz modulierten zweiten Messsignalanteil (M2) umfasst wobei
der Kraft- und Temperatursensor (12; 22; 32; 42; 54) einen Schwingkörper (17; 27; 37; 47; 58) und Mittel zur Übertragung des Auslesesignals zum Schwingkörper und zum Versetzen desselben in Schwingungen mit der Trägerfrequenz umfasst.

2. Messeinrichtung nach Anspruch 1, **dadurch gekennzeichnet, dass** der Kraft- und Temperatursensor (12; 22; 32; 42; 54) vom FBG-Typ oder Fabry-Perot-Typ ist.

3. Katheteranordnung (10; 20; 30; 40), **dadurch gekennzeichnet, dass** sie aufweist:
- eine Messeinrichtung nach einem der Ansprüche 1 oder 2, wobei die Messeinrichtung in einen distalen Katheterabschnitt (11.1; 21.1; 31.1; 41.1; 51) integriert ist und zugleich derart ausgebildet und angeordnet ist, dass sie die im distalen Katheterabschnitt herrschende Temperatur annimmt, und
- eine an den Kraft- und Temperatursensor der Messeinrichtung angeschlossene Auswertungseinrichtung,
- wobei die Auswerteeinrichtung weiter aufweist:
- einen Auslesesignalgenerator zur Erzeugung und Einspeisung eines mit einer Trägerfrequenz modulierten Auslesesignals in den Kraft- und Temperatursensor,
- erste Auslesemittel (18.1; 28.1; 38.1; 48.1) zum Auslesen eines mit der Trägerfrequenz modulierten ersten Messsignalanteils (M1),
- zweite Auslesemittel (18.2; 28.2; 38.2; 48.2) zum Auslesen eines nicht mit der Trägerfrequenz modulierten zweiten Messsignalanteils (M2), sowie
- Verarbeitungsmittel (19; 29; 39; 49) zur kombinierten Verarbeitung des ersten und zweiten Messsignalanteils zur Bestimmung einer ausschließlich kraft- bzw. dämpfungsabhängigen Komponente und einer ausschließlich temperaturabhängigen Komponente des Sensorsignals.

4. Katheteranordnung nach Anspruch 3, **dadurch gekennzeichnet, dass** die Mittel zum Übertragen des Auslesesignals einen Antriebsdraht (17a; 53) umfassen, der bis zum proximalen Ende (11.2; 51) des Katheters verläuft.

5. Katheteranordnung nach Anspruch 3, **dadurch gekennzeichnet, dass** die Mittel zum Übertragen des Auslesesignals eine elektrische Leitung (25a), die sich bis zu einem Anschluss am proximalen Ende (21.2) des Katheters erstreckt, und einen elektromechanischen oder Piezo-Wandler (26) umfassen.

6. Katheteranordnung nach Anspruch 3, **dadurch gekennzeichnet, dass** die Mittel zum Übertragen des Auslesesignals eine sich bis zum proximalen Ende des Katheters (41.2) erstreckende Flüssigkeitsleitung, insbesondere einen Spülkanal (46), und einen hydraulischen Wandler (46b) umfassen.

7. Katheteranordnung nach Anspruch 3, **dadurch gekennzeichnet, dass** die Mittel zum Übertragen des Auslesesignals eine sich bis zum proximalen Ende des Katheters (31.2) erstreckende Lichtleitfaser (35b) und einen dem Schwingkörper zugeordneten opto-mechanischen Wandler (36) umfassen.

8. Katheteranordnung nach einem der Ansprüche 3 bis 7, **dadurch gekennzeichnet, dass** die zweiten Auslesemittel (18.2) zum Auslesen eines ummodulierten zweiten Messsignalanteils (M2) ausgebildet sind.

9. Katheteranordnung nach einem der Ansprüche 3 bis 8, **dadurch gekennzeichnet, dass** die Mittel zum Übertragen des Auslesesignals eine elektrische Leitung (25a), die sich bis zu einem Anschluss an dem proximalen Ende (21.2) des Katheters erstreckt, und einen dem Schwingkörper (27) zugeordneten elektromechanischen oder Piezo-Wandler (26), oder eine sich bis zum proximalen Ende (41.2) des Katheters erstreckende Flüssigkeitsleitung, insbesondere Spülflüssigkeitsleitung (46), und einem dem Schwingkörper (47) zugeordneten hydraulischen Wandler (46a), oder eine sich bis zum proximalen Ende des Katheters (31.2) erstreckende Lichtleitfaser (35b) und einen dem Schwingkörper (37) zugeordneten opto-mechanischen Wandler (36), umfassen.

10. Katheteranordnung nach einem der Ansprüche 3 bis 9, ausgebildet als Ablationskatheter (11; 21; 31; 41).

## Claims

1. A measuring device for force and/or attenuation measurements (54), comprising an individual combined force and temperature sensor (12; 22; 32; 42; 54) and input means (16, 17a; 25a, 26; 35b, 36; 46a, 46, 46b; 53) for feeding a readout signal modulated with a carrier frequency and output means (13; 23; 33; 43; 55) for outputting a measuring signal, which comprises a first measuring signal portion (M1) modulated with the carrier frequency and a second measuring signal portion (M2) not modulated with the carrier frequency,
wherein
the force and temperature sensor (12; 22; 32; 42; 54) comprises an oscillating body (17; 27; 37; 47; 58) and means for transmitting the readout signal to the oscillating body and for oscillating said oscillating body with the carrier frequency.

2. The measuring device according to Claim 1, **characterised in that** the force and temperature sensor (12; 22; 32; 42; 54) is of the FBG type or Fabry-Perot type.

3. A catheter arrangement (10; 20; 30; 40), **characterised in that** it comprises:
- a measuring device according to one of Claims 1 or 2, wherein the measuring device is integrated in a distal catheter portion (11.1;21.1;31.1;41.1;51) and is simultaneously configured and arranged in such a way that it receives the temperature prevailing in the distal catheter portion, and
- an evaluation device connected to the force and temperature sensor of the measuring device,
- wherein the evaluating device further comprises:
- a readout signal for generating and feeding a readout signal modulated with a carrier frequency in the force and temperature sensor,
- first readout means (18.1; 28.1; 38.1; 48.1) for reading out a first measuring signal portion (M1) modulated with the carrier frequency,
- second readout means (18.2; 28.2; 38.2; 48.2) for reading out a second measuring signal portion (M2) not modulated with the carrier frequency, and
- processing means (19; 29; 39; 49) for combined processing of the first and second measuring signal portion in order to determine an exclusively force- or attenuation-dependent component and an exclusively temperature-dependent component of the sensor signal.

4. The catheter arrangement according to Claim 3, **characterised in that** the means for transmitting the readout signal comprise a drive wire (17a; 53), which runs as far as the proximal end (11.2; 51) of the catheter.

5. The catheter arrangement according to Claim 3, **characterised in that** the means for transmitting the readout signal comprise an electrical line (25a), which extends as far as a connection at the proximal end (21.2) of the catheter, and an electromechanical or piezo-transducer (26).

6. The catheter arrangement according to Claim 3, **characterised in that** the means for transmitting the readout signal comprise a liquid line, in particular an irrigation duct (46) extending as far as the proximal end of the catheter (41.2), and a hydraulic transducer (46b).

7. The catheter arrangement according to Claim 3, **characterised in that** the means for transmitting the readout signal comprise an optical fibre (35b) extending as far as the proximal end of the catheter (31.2) and an opto-mechanical transducer (36) associated with the oscillating body.

8. The catheter arrangement according to one of Claims 3 to 7, **characterised in that** the second readout means (18.2) are configured to read out an unmodulated second measuring signal portion (M2).

9. The catheter arrangement according to one of Claims 3 to 8, **characterised in that** the means for transmitting the readout signal comprise an electrical line (25a), which extends as far as a connection at the proximal end (21.2) of the catheter, and an electromechanical or piezo-transducer (26) associated with the oscillating body (27), or a liquid line, in particular an irrigation liquid line (46), extending as far as the proximal end (41.2) of the catheter, and a hydraulic transducer (46a) associated with the oscillating body (47), or an optical fibre (35b) extending as far as the proximal end of the catheter (31.2) and an opto-mechanical transducer (36) associated with the oscillating body (37).

10. The catheter arrangement according to one of Claims 3 to 9, configured as an ablation catheter (11; 21; 31; 41).

## Revendications

1. Dispositif de mesure pour des mesures de forces et/ou d'atténuations (54), avec un unique capteur optique de température et de force combiné (12; 22; 32; 42; 54) et des moyens d'entrée (16, 17a; 25a, 26; 35b, 36; 46a, 46, 46b; 53) pour l'alimentation d'un signal de lecture modulé avec une fréquence porteuse, et des moyens de sortie (13; 23; 33; 43; 55) pour la délivrance d'un signal de mesure, lequel comprend une première composante de signal de mesure (M1) modulée avec une fréquence porteuse et une deuxième composante de signal de mesure (M2) non modulée avec la fréquence porteuse,
dans lequel
le capteur de température et de force (12; 22; 32; 42; 54) comprend un corps oscillant (17; 27; 37; 47; 58) et des moyens pour transmettre le signal de lecture au corps oscillant et pour faire osciller celui-ci avec la fréquence porteuse.

2. Dispositif de mesure selon la revendication 1, **caractérisé en ce que** le capteur de température et de force (12; 22; 32; 42; 54) est du type FBG ou du type Fabry-Perot.

3. Ensemble de cathéter (10; 20; 30; 40) **caractérisé en ce qu'**il comporte:
- un dispositif de mesure selon l'une des revendications 1 ou 2, le dispositif de mesure étant intégré dans une section distale de cathéter (11.1; 21.1; 31.1; 41.1; 51) tout en étant conçu et agencé de manière à prendre la température régnant dans la section distale de cathéter, et
- un dispositif d'évaluation raccordé au capteur de température et de force du dispositif de mesure,
- dans lequel le dispositif d'évaluation comprend en outre :
- un générateur de signaux de lecture pour la production et l'alimentation d'un signal de lecture modulé avec la fréquence porteuse vers le capteur de température et de force,
- des premiers moyens de lecture (18.1; 28.1; 38.1; 48.1) pour la lecture d'une première composante de signal de mesure (M1) modulée avec la fréquence porteuse,
- des deuxièmes moyens de lecture (18.2; 28.2; 38.2; 48.2) pour la lecture d'une deuxième composante de signal de mesure (M2) non modulée avec la fréquence porteuse, et
- des moyens de traitement (19; 29; 39; 49) pour le traitement combiné de la première et de la deuxième composante de signal de mesure, en vue de la détermination d'une composante du signal de capteur exclusivement dépendante des forces et atténuations et d'une composante exclusivement dépendante de la température.

4. Ensemble de cathéter selon la revendication 3, **caractérisé en ce que** les moyens pour la transmission du signal de lecture comprennent un fil d'entraînement (17a; 53) s'étendant jusqu'à l'extrémité proximale (11.2; 51) du cathéter.

5. Ensemble de cathéter selon la revendication 3, **caractérisé en ce que** les moyens pour la transmission du signal de lecture comprennent une ligne électrique (25a) s'étendant jusqu'à un raccord à l'extrémité proximale (21.2) du cathéter, ainsi qu'un transducteur électromécanique ou piézoélectrique (26).

6. Ensemble de cathéter selon la revendication 3, **caractérisé en ce que** les moyens pour la transmission du signal de lecture comprennent une conduite de liquide s'étendant jusqu'à l'extrémité proximale du cathéter (41.2), de préférence un canal de rinçage (46), et un convertisseur hydraulique (46b).

7. Ensemble de cathéter selon la revendication 3, **caractérisé en ce que** les moyens pour la transmission du signal de lecture comprennent une fibre optique (35b) s'étendant jusqu'à l'extrémité proximale du cathéter (31.2), ainsi qu'un convertisseur opto-mécanique (36).

8. Ensemble de cathéter selon l'une des revendications 3 à 7, **caractérisé en ce que** les deuxièmes moyens de lecture (18.2) sont conçus pour la lecture d'une deuxième composante de signal de mesure non modulée (M2).

9. Ensemble de cathéter selon l'une des revendications 3 à 8, **caractérisé en ce que** les moyens pour la transmission du signal de lecture comprennent une ligne électrique (25a) s'étendant jusqu'à un raccord à l'extrémité proximale (21.2) du cathéter, ainsi qu'un transducteur électromécanique ou piézoélectrique (26) attribué au corps oscillant (27), ou une conduite de liquide s'étendant jusqu'à l'extrémité proximale (41.2) du cathéter, en particulier une conduite de liquide de rinçage (46), et un convertisseur hydraulique (46a) attribué au corps oscillant (47), ou une fibre optique (35b) s'étendant jusqu'à l'extrémité proximale du cathéter (31.2) et un convertisseur opto-mécanique (36) attribué au corps oscillant (37).

10. Ensemble de cathéter selon l'une des revendications 3 à 9, conçu comme un cathéter d'ablation (11; 21; 31; 41).
